# EUROPEAN PATENT APPLICATION

(11) **EP 3 135 299 A1**
(43) Date of publication of application: **01.03.2017**
(21) Application number: 15782882.3
(22) Date of filing: 22.04.2015
(51) Int. Cl.: A61K 47/34, A61K 47/30, A61K 9/48, A61K 31/5377

(54) **ACTIVE INGREDIENT (I) CONTAINING COMPOSITION AND METHOD FOR PREPARING SAME**

(30) Priority: 22.04.2014 KR 20140048064
(71) Applicant: SK Chemicals Co., Ltd., Seongnam-si, Gyeonggi-do 463-400 (KR)
(72) Inventor: PARK, Jun-sung, Seongnam-si Gyeonggi-do 463-916 (KR); SHIN, DongChul, Yongin-si Gyeonggi-do 446-878 (KR); RYU, Keun-Ho, Seoul 151-770 (KR); SHIN, Ho Chul, Seongnam-si Gyeonggi-do 463-748 (KR); HWANG, Sang-wook, Yongin-si Gyeonggi-do 448-784 (KR); KIM, Gwan-young, Yongin-si Gyeonggi-do 446-741 (KR); KIM, Hun-Taek, Seoul 137-951 (KR)
(74) Representative: Graf von Stosch, Andreas
(86) International application number: PCT/KR2015/004021
(87) International publication number: WO 2015/163689

(57) **Abstract**

The present invention relates to a composition comprising 5-chloro-N-({(5S)-2-oxo-3-[4-(3-oxo-4-morpholinyl)-phenyl]-1,3-oxazolidin-5-yl}-methyl]-2-thiophene carboxyamide as an active ingredient, manufactured by a melt extrusion method. The composition according to the present invention has a wide variety of advantages such as securing dissolution rate and bioavailability of the active ingredient, improving absorption deviation depending on whether a patient has a meal or not, and securing stability during the manufacturing process. The present invention further provides a method which can manufacture the said composition.

## Description

### TECHNICAL FIELD

The present application claims priority to Korean Patent Application No. 10-2014-0048064 filed on April 22, 2014 in the Republic of Korea, the disclosures of which are incorporated herein by reference.

The present disclosure relates to a solid composition for oral administration comprising 5-chloro-N-({(5S)-2-oxo-3-[4-(3-oxo-4-morpholinyl)-phenyl]-1,3-oxazolidin-5-yl}-methyl]-2-thiophenecarboxyamide (Hereinafter, active ingredient (Form I), active ingredient (I) or rivaroxaban) in the form of amorphous and/or thermodynamically metastable crystal, which is manufactured by a melt extrusion method, orally administered, rapidly releases the active ingredient (I), and, in particular, has improved stability on a melt extrusion manufacturing process, and a method for manufacturing thereof.

### BACKGROUND ART

The active ingredient (Form I )(Active ingredient (I) or rivaroxaban) of the following formula is a drug for oral administration, and a low molecular weight inhibitor of coagulation factor Xa, which can be used for various thromboembolic diseases and their secondary treatment and/or prevention (International Patent Publication No. WO2001/147919).

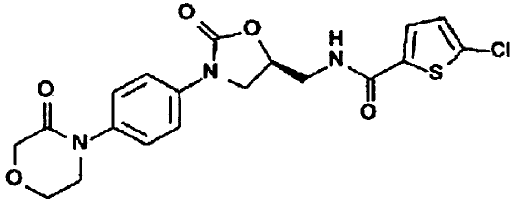

Commercially available active ingredient (I) is designed as a formulation, which can reach 100% bioavailability regardless of a meal when administered at 10 mg. However, it can be found that in the case at 20 mg, there is a burden to have to take it during a meal. At single administration and repetitive administration, there is a difference in blood concentration depending on whether taking food or not, and food intake is blamed for causing the difference (Xia et al., British Journal of Clinical Pharmacology, 2009, 68:1, p77-88). This is a reason for decrease of drug efficacy to patients taking food irregularly, and a negative cause, which may make patients feel uncomfortable for taking drug. This is caused by difference in solubility of the active ingredient (I), and in the 10 mg of the active ingredient (I) having solubility in water (37°C) of 9 µg/ml, 90% or more of the drug is dissolved in the volume of a generally known dissolution solution (900 ml). On the other hand, 20 mg is a condition that just 50% of the drug is dissolved, and at this condition, its bioavailability is about 66% when it is administered before a meal. Thus, its bioavailability reach to 100% when it is administered with food because residual insoluble drugs are absorbed by oil content present in the food and a surfactant such as bile acid secreted in the body (Samama et al. Thrombosis Journal 2013, 11:11, p1-7).

Thus, in order to overcome these problems, it is needed to manufacture a drug to have higher bioavailability when it is administered regardless of food intake by solubilization of the active ingredient (I), thereby providing higher convenience for drug intake to patients. As a result, it is essential to develop a formulation having higher drug compliance.

Various methods are tried to solubilize this poorly water soluble active ingredient (I), i.e., rivaroxaban.

International Patent Publication No.WO2010/003641 discloses a melt-extruded formulation comprising all three of an active ingredient (I), a surfactant, and a pseudo-emulsifier as an essential ingredient. However, it is difficult to obtain the level of solubility and crystal change desired in the present invention at the exemplary process condition of 70∼160°C.

Further, International Patent Publication No.WO2014/016842 discloses a method for solubilizing the active ingredient (I) by manufacturing it as an amorphous form. Specifically, it mentions a method wherein an excipient, preferably hypromellose phthalate and the active ingredient (I) are dissolved in a solvent and then the excipient and the active ingredient (I) are coprecipitated by evaporating the solvent to manufacture the amorphous form. This method is the most common method used for manufacturing an amorphous solid dispersion, but use of a toxic organic solvent may be unavoidable, an additional cost may be required for use of an organic solvent, and there may be a safety problem when a residual solvent is remained in the coprecipitate. Thus, there is a disadvantage that an additional drying process should be included in the manufacturing process.

Further, in International Patent Publication No. WO2011/042156 a composition was manufactured by melt extrusion using the active ingredient (I), a water soluble polymer, and a disintegrating agent as an essential ingredient, and adding a material, which forms pores in the granule as needed thereby enabling a dissolution solution to be penetrated into the granule. In this patent, process temperature is controlled to 90∼160°C so that the form of the active ingredient (I) is maintained in the crystal form. Namely, the granule is manufactured not to contain any amorphous active ingredient (I) in it, and this displays a clear difference from the present invention, which is objected to provide a pharmaceutical formulation comprising an amorphous and/or thermodynamically metastable active ingredient (I). In addition, when using an unmolten material such as a disintegrating agent in the melt extrusion method, there is a high risk to cause nonuniformity of mixing, particularly during mass production.

Further, International Patent Publication No. WO2007/039122 discloses the active ingredient (I) in the amorphous and/or thermodynamically metastable crystal form. In particular, in manufacture of the amorphous form by a melting method and a melt extrusion method, this patent suggests the result of using polyethylene glycol (PEG6000) in the melting method, hydroxypropylcellulose (HPC) and polyvinylpyrrolidone (PVP) was used to confirm bioavailability improvement in the manufacture by the melt extrusion method. In particular, the patent used a similar melting method and melt extrusion method with the present invention, but there is no description about a specific process temperature range, which is necessary for amorphization of the active ingredient (I). Further, in the detailed description, the amount ratio of the active ingredient (I) and the water soluble polymer is 1:9, and in order to make a formulation such as tablet from them, a large amount of additional excipients should be added, and this cause increase of tablet size. Therefore, this may reduce intake convenience of patients.

### DISCLOSURE

### Technical Problem

The present disclosure is designed to solve the problems of the related art, and therefore the present disclosure is directed to providing a rivaroxaban-containing composition having a wide variety of advantages such as securing bioavailability of the active ingredient, improving absorption deviation depending on whether a patient has a meal or not, and securing stability during the manufacturing process, and a method for manufacturing thereof.

These and other objects and advantages of the present disclosure may be understood from the following detailed description and will become more fully apparent from the exemplary embodiments of the present disclosure. Also, it will be easily understood that the objects and advantages of the present disclosure may be realized by the means shown in the appended claims and combinations thereof.

### Technical Solution

In one aspect of the present disclosure, there is provided a rivaroxaban-containing composition manufactured by a melt extrusion method using rivaroxaban and at least one pharmaceutically acceptable polymer, wherein the rivaroxaban is in the amorphous and/or thermodynamically metastable crystal form, and the pharmaceutically acceptable polymer is vinylpyrrolidone-vinylacetate copolymer, polyethylene glycol-polyvinylcaprolactam-polyvinylacetate copolymer, or a mixture thereof.

In the composition of the present invention, the rivaroxaban and at least one pharmaceutically acceptable polymer exist in the form of uniformly mixed solid dispersion, the rivaroxaban exists in the form of amorphous and/or thermodynamically metastable crystal morphosis in a pharmaceutical formulation, and other additives such as a surfactant may be further contained in this solid dispersion within the range not hindering the purpose of the present invention.

Preferably, about 80 weight% or more, more preferably, about 90 weight% or more, further more preferably, about 95 weight% or more, the most preferably, about 99 weight% or more of the rivaroxaban based on its total weight exists in the amorphous form.

The polymer for manufacturing the solid dispersion of the present invention may be vinylpyrrolidone-vinylacetate copolymer (for example, PVPVA64), polyethylene glycol-polyvinylcaprolactam-polyvinylacetate copolymer (for example, Soluplus) or a mixture thereof, and only such polymer(s) could efficiently achieve the purpose of the present invention such as enhancing dissolution rate, securing content uniformity, securing easy of manufacture, securing drug stability during manufacture, and the like.

The present invention developed a composition enabling drug intake regardless of a meal. The rivaroxaban-containing composition according to the present invention can display release of 80% (preferably 85%, more preferably 90%) or more of the active ingredient (I) within 2 hours in various *in vitro* experiments, in particular, a dissolution solution not containing a surfactant, representatively, distilled water. Further, considering based on the result of *in vivo* experiment, the drug can be taken regardless a meal because solubilization enhanced bioavailability, and therefore, it is available to remove various impacts by patients or caused by patient. In addition, when using the melt extrusion method, which can make a uniform mixture by complete melting of the mixture, the composition can be manufactured by excluding the influence of the initial particle size of the active ingredient (I); the composition having uniform distribution of the active ingredient (I) can be manufactured; a separate drying process is not required because a solvent is not used; when manufacturing tablet and capsule using the minimum amount of water soluble polymer, a formulation having the size not burdening patients can be designed, thereby increasing drug compliance of patients; and because the formulation can be manufactured by using direct tableting through simple capsule filling and simple mixing, convenience on process may also be largely increased. Further, there is a large advantage that stability of the rivaroxaban can be secured when manufacturing the composition according to the present invention by using the melt extrusion method.

Preferably, the rivaroxaban contained in the composition according to the present invention has solubility of 18 µg/ml or more in 37°C distilled water.

Preferably, in the rivaroxaban-containing composition according to the present invention, the polymer includes vinylpyrrolidone-vinylacetate copolymer. In the vinylpyrrolidone-vinylacetate copolymer, ratio of vinylpyrrolidone and vinylacetate may preferably be 60:40 (vinylpyrrolidone : vinylacetate), but not limited thereto. If the polymer is vinylpyrrolidone-vinylacetate copolymer, it is more preferred to the purpose of the present invention such as dissolvability, stability and the like.

Preferably, in the molten extrudate according to the present invention, ratio of the polymer is at least 30 weight% of the total weight of the molten extrudate. Because the polymer additionally used has higher solubility as its amount is increased, the ratio can be controlled until a mixture having the desired level of solubility is obtained. Thus, preferably, in the composition according to the present invention, content (weight) ratio of the rivaroxaban and the polymer is 1:0.5 to 1:5 (rivaroxaban : total polymer content).

Preferably, in order to achieve the purpose according to the present invention, the melt extrusion should be conducted at 160 to 230°C, more preferably at 180 to 210°C. When it is conducted at this temperature, the rivaroxaban contained in the composition becomes amorphous or thermodynamically metastable crystal form, and at the same time, stability of the rivaroxaban can be secured.

In the present invention, the temperature of the melt extrusion means the maximum temperature on the surface of the melt extrusion device to which the mixture of the rivaroxaban and the polymer contacts on the process.

As mentioned above, the composition according to the present invention may comprise other additional ingredients other than the rivaroxaban and the polymer in the molten extrudate within the range not hindering the purpose of the present invention, and for example, the molten extrudate is manufactured by mixing a surfactant in order to additionally increase solubility of the active ingredient (I).

Preferably, this surfactant is contained in an amount of 30 weight% or less, preferably 20% or less, more preferably 10% or less, the most preferably 5% or less, based on the total weight of the rivaroxaban and the polymer.

For example, this surfactant may be a non-ionic surfactant such as poloxamers, polyethoxylated castor oils (Cremophor), vitamin E-TPGS and lauroyl polyoxylglycerides (Gelucire), an anionic surfactant such as sodium dodecyl sulfate and the like, but the present invention is not limited to these surfactant types.

Preferably, 60% or more, 70% or more, preferably 80% or more, more preferably 85% or more of the rivaroxaban in the composition according to the present invention is released within 2 hours at a condition of rotating a paddle at 75 rpm and using water (preferably, distilled water) 900 ml as a dissolution media (other condition follows dissolution method 2 of U.S. Pharmacopoeia), and more preferably, this active ingredient (I) is not precipitated until 2 hour or 4 hour dissolution is completed, and maintains dissolution rate.

The extrudate, which comprises the active ingredient (I) according to the present invention and is obtained by the melt extrusion method, can be optionally cut, rounded out or coated as needed, processed to, for example, sachet formulation, or filled into a capsule. When it is manufactured as capsule, it can be mixed with other additives commonly mixed for manufacturing capsule before filling the capsule. Further, the molten extrudate can be micronized by using an air jet mill, a ball-mill or a high pressure homogenizer, mixed with common additives used for manufacturing tablet (for example, disintegrating agent, excipient, lubricant and the like), and then pressed as a tablet.

Further, the present invention provides a method for manufacturing the composition comprising the rivaroxaban in the amorphous and/or thermodynamically metastable crystal form, which is characterized by comprising the steps of: (S1) mixing (a) the rivaroxaban and (b) the polymer selected from vinylpyrrolidone-vinylacetate copolymer, polyethylene glycol-polyvinylcaprolactam-polyvinylacetate copolymer, or a mixture thereof (preferably, vinylpyrrolidone-vinylacetate copolymer), and (S2) melt extruding the mixture.

Preferably, the manufacturing method according to the present invention is characterized that content (weight) ratio of the rivaroxaban and the polymer is 1:0.5 to 1:5 (rivaroxaban : total polymer content).

Preferably, in the manufacturing method according to the present invention, the melt extrusion is conducted at 160 to 230°C, more preferably at 180 to 210°C.

In the manufacturing method according to the present invention, optionally, other additives (for example, surfactant) other than the rivaroxaban and the polymer may be added to the mixture of the (S1) step within the range not hindering the purpose of the present invention, and the molten extrudate may go through additional processing steps such as cutting, coating and the like after melt extrusion. Further, in order to manufacture capsule, tablet and the like, the molten extrudate can be mixed with other additives commonly added to these formulations (for example, excipient, lubricant, filler, disintegrating agent).

### Advantageous Effects

The present disclosure gives the following effects.

The present invention provides a rivaroxaban-containing composition which has various advantages such as securing dissolution rate and bioavailability, improving absorption deviation depending on whether a patient has a meal or not, and securing stability during a manufacturing process, and a method for manufacturing thereof.

### DESCRIPTION OF DRAWINGS

Other objects and aspects of the present disclosure will become apparent from the following descriptions of the embodiments with reference to the accompanying drawings in which:
The accompanying drawings illustrate a preferred embodiment of the present disclosure and together with the foregoing disclosure, serve to provide further understanding of the technical spirit of the present disclosure, and thus, the present disclosure is not construed as being limited to the drawing.
   Fig. 1 is the result of comparative evaluation of dissolution in distilled water (37°C, 900 ml, 75 rpm) for Examples according to the present invention and Comparative Example 2 (Xarelto 20mg tablet).
   Fig. 2 is the PXRD result of the micronized active ingredient (I) of Comparative Example 1.
   Fig. 3 is the PXRD result of Example 12.
   Fig. 4 the PXRD result of Example 16.
   Fig. 5 the PXRD result of Example 22.
   Fig. 6 is a graph showing blood (plasma) concentration change after intaking Comparative Example 2 and Example 16 as an example according to the present invention.

### BEST MODE

Hereinafter, preferred embodiments of the present disclosure will be described in detail with reference to the accompanying drawings. Prior to the description, it should be understood that the terms used in the specification and the appended claims should not be construed as limited to general and dictionary meanings, but interpreted based on the meanings and concepts corresponding to technical aspects of the present disclosure on the basis of the principle that the inventor is allowed to define terms appropriately for the best explanation. Therefore, the description proposed herein is just a preferable example for the purpose of illustrations only, not intended to limit the scope of the disclosure, so it should be understood that other equivalents and modifications could be made thereto without departing from the scope of the disclosure.
Comparative Example 1: Micronized Raw Material Particle of Active Ingredient (I) (Rivaroxaban) (Particle size (d90) corresponding to 90% of the maximum particle size in particle size cumulative distribution is 15 µm or less)
Comparative Example 2: Xarelto^{™} 20 mg Tablet
Comparative Example 3: Formulation 2 of International Patent Publication No. WO2007/039122 (Active ingredient (I) + Hydroxypropylcellulose (HPC) + Xylitol)
Comparative Example 4: Formulation 3 of International Patent Publication No. WO2007/039122 (Active ingredient (I) + Polyvinylpyrrolidone (PVP) + Xylitol)

### Test Example 1 : Solubility Test (Distilled water, 37°C, 24 hour)

- Test Method: Solid dispersion corresponding to about 20 mg of active ingredient (I) was put into 200 ml distilled water, stored at 37°C, 100 rpm for 24 hours, and then solubility of the drug was observed. Solid dispersion corresponding to 5 mg of active ingredient (I) contained a large amount of copolymer and a small amount of surfactant, but it was confirmed by this test that the amount level does not affect to solubility of the drug. The process temperature specified in the following Example means the maximum temperature on the surface of the melt extrusion device to which the mixture contacts on the process.

**Table 1**

| Solubility of Comparative Example 1 | |
|---|---|
| | Comparative Example 1 |
| Solubility (37°C, 24 hour) | 9.86 µg/ml |

**Table 2**

| Solubility of Comparative Example 1 at various pH | | | | | |
|---|---|---|---|---|---|
| Unit: µg/ml | pH1.2 | pH4.0 | pH6.0 | pH6.8 | pH7.4 |
| Solubility (37°C, 24 hour) | 10.77 | 10.89 | 10.34 | 8.06 | 8.69 |

**Table 3**

| Vinylpyrrolidone-vinylacetate copolymer (PVPVA64): Solubility according to various mixing ratio of rivaroxaban (process temperature 230°C) | | | | | |
|---|---|---|---|---|---|
| Unit: µg/ml | 0.5:1 Example 1 | 1:1 Example 2 | 2:1 Example 3 | 3:1 Example 4 | 5:1 Example 5 |
| Solubility (37°C, 24 hour) | 28.58 | 33.10 | 45.41 | 52.22 | 47.50 |

**Table 4**

| PVPVA64: Solubility change of Rivaroxaban (2:1) composition according to process temperature | | | | | |
|---|---|---|---|---|---|
| Unit: µg/ml | 180°C Example 6 | 190°C Example 7 | 200°C Example 8 | 210°C Example 9 | 230°C Example 10 |
| Solubility (37°C, 24 hour) | 17.73 | 21.15 | 26.52 | 39.75 | 45.41 |

**Table 5**

| PVPVA64: Mixture of several weight% of Cremophor A25 based on rivaroxaban (3:1) (Process temperature 210°C) | | |
|---|---|---|
| Unit: µg/ml | 0% mix Example 11 | 5% mix Example 12 |
| Solubility (37°C, 24 hour) | 37 | 37 |

**Table 6**

| PVPVA64: Solubility according to process temperature of mixture of 5 weight% of vitamin E-TPGS based on rivaroxaban (3:1) | | | | |
|---|---|---|---|---|
| Unit: µg/ml | 180°C Example 13 | 190°C Example 14 | 200°C Example 15 | 210°C Example 16 |
| Solubility (37°C, 24 hour) | 39.60 | 41.69 | 46.98 | 47.75 |

**Table 7**

| Polyethylene glycol-polyvinylcaprolactam-polyvinylacetate copolymer (Soluplus): Solubility according to process temperature of mixture of 5 weight% of vitamin E-TPGS based on rivaroxaban (3:1) | | | | |
|---|---|---|---|---|
| Unit: µg/ml | 180°C Example 17 | 190°C Example 18 | 200°C Example 19 | 210°C Example 20 |
| Solubility (37°C, 24 hour) | 24.43 | 28.86 | 38.80 | 33.07 |

**Table 8**

| PVPVA64: Solubility according to process temperature of rivaroxaban (5:1) mixture | | |
|---|---|---|
| Unit: µg/ml | 200°C Example 21 | 210°C Example 22 |
| Solubility (37°C, 24 hour) | 45.85 | 50.21 |

### Test Example 2: Dissolution Test (Distilled water 900 ml)

- Test Method: a solid dispersion (20 mg as rivaroxaban) manufactured in 37°C distilled water (900 ml) by a melt extrusion method was put into a dissolution solution, and then degree of drug release was evaluated while rotating the solution at 75 rpm (paddle) (other condition follows dissolution method 2 of U.S. Pharmacopoeia).

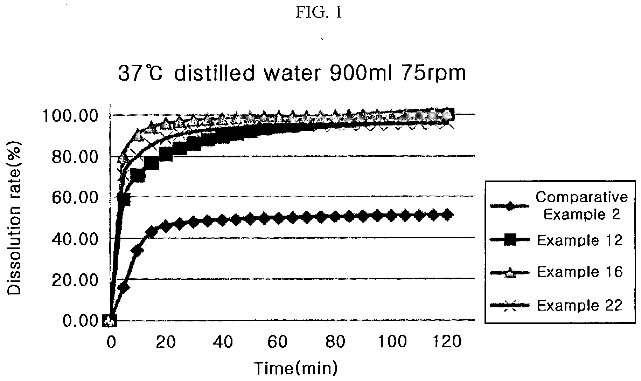

The results are shown in Fig. 1. As shown in Fig. 1, it can be confirmed that the solubilized solid dispersions showed difference in the initial dissolution rate in distilled water 900 ml, but all of them showed dissolution rate of 85% or more after 30 min.

### Test Example 3: Evaluation of Crystal Form

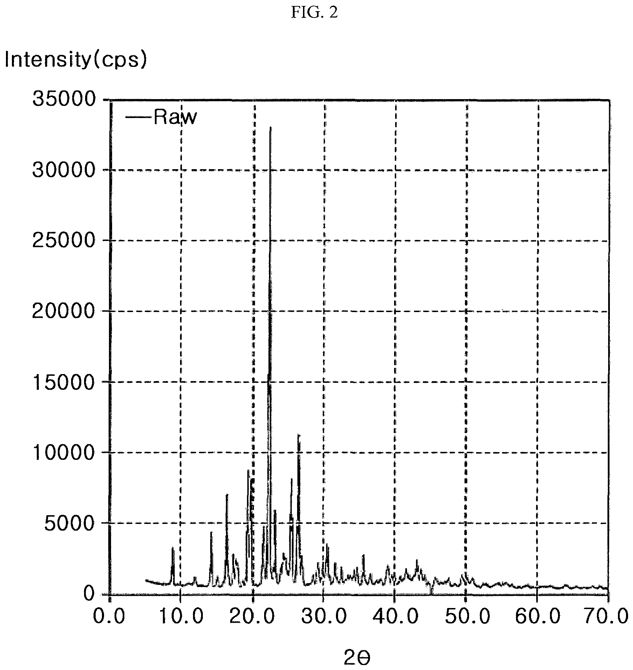

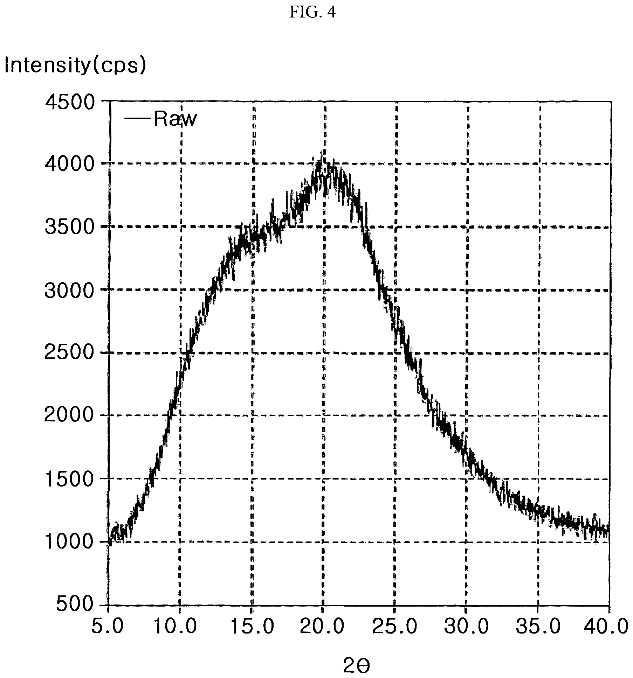

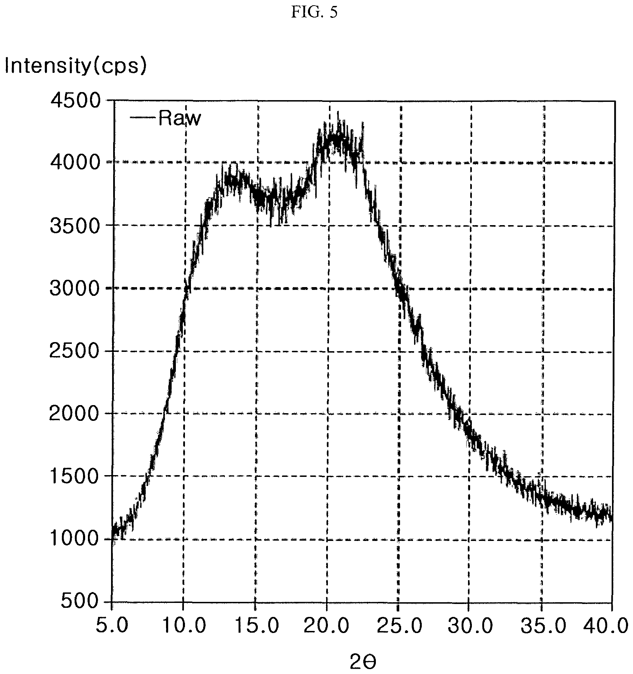

Through crystal form analysis for one Example according to the present invention, whether the active ingredient (I) is in the form of amorphous and/or thermodynamically metastable crystal or not was checked. The results were shown in Figs. 2 to 5.

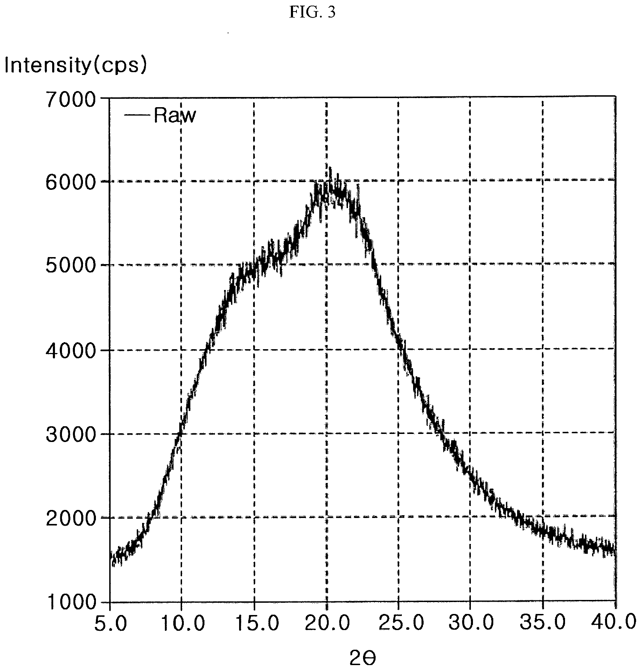

As shown in Figs. 3 to 5, it was confirmed that the active ingredient (I) exists in the amorphous and/or thermodynamically metastable crystal form at various process conditions and compositions.

### Test Example 4: Comparison of Stability by Comparing Production Amount of Impurities

It can be confirmed that two impurities are generated when manufacturing the rivaroxaban-containing composition using the melt extrusion method according to the present invention. Those can be classified into 0.50 (Impurity A) and 1.29 (Impurity B) according to Relative Retention Time (RRT). It was confirmed that Comparative Examples 3 and 4 generate more impurities than Example 11 when manufacturing at the same process and condition. The results were shown in the following Table 9.

**Table 9**

| | Comparative Example 3 | Comparative Example 4 | Example 11 |
|---|---|---|---|
| Impurity A | 1.01% | 2.08% | 0.17% |
| Impurity B | 1.66% | 0.29% | 0.20% |

From the results of the above Table 9, it can be found that at the same melt extrusion process condition, PVPVA64 is more preferable polymer to the melt extrusion process because it generates less impurities than HPC and PVP. In particular, in the case of high generation of impurities of 1 % or more, safety should be verified by proceeding qualification for the impurities in accordance with ICH guideline, and furthermore, there is a high possibility that the case will be insufficient to be used as a commercial medicine.

### Test Example 5: Pharmacokinetic Evaluation

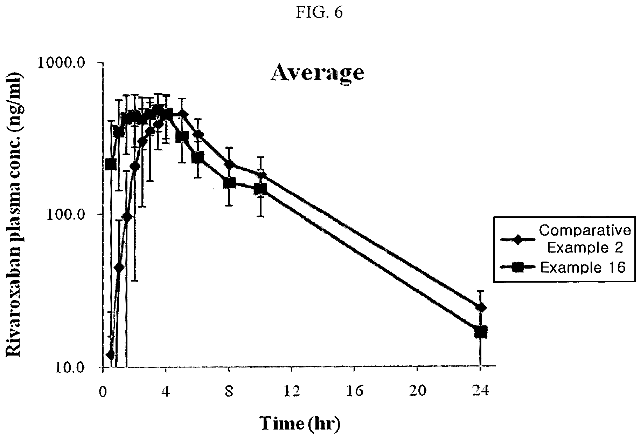

Pharmacokinetic evaluation was conducted for the formulations manufactured from Comparative Example 2 and Example 16. Patients were divided into two group (three patients per group), and Comparative Example 2 was administered between meals and Example 16 was administered under fasted condition. Then, blood was collected according to the given distance of time, and pharmacokinetic profiles were confirmed, respectively. The results were shown in the following Fig. 6 and Table 10. For reference, because Comparative Example 2 containing the active ingredient (I) in an amount of 20 mg shows low bioavailability of 66% when administered under fasted condition, it is strongly recommended to always administer it with food (The Journal of Clinical Pharmacology, 46, 549-558, 2006).

**Table 10**

| | Cₘₐₓ (ng/ml) | AUCₗₐₛₜ (hr*ng/ml) |
|---|---|---|
| Comparative Example 2 | 516.5 | 4048.4 |
| Example 16 | 559.2 | 4020.9 |

From the results of the above Fig. 6 and Table 10, it was confirmed that Example 16 of the solubilized active ingredient (I) has Cₘₐₓ and AUC equal to those of Comparative Example 2, which should be administered with food, although it was administered under fasted condition.

The present disclosure has been described in detail. However, it should be understood that the detailed description and specific examples, while indicating preferred embodiments of the disclosure, are given by way of illustration only, since various changes and modifications within the scope of the disclosure will become apparent to those skilled in the art from this detailed description.

## Claims

1. A rivaroxaban-containing composition manufactured by a melt extrusion method using rivaroxaban and at least one pharmaceutically acceptable polymer,
wherein the rivaroxaban is in the amorphous or thermodynamically metastable crystal form, and
the pharmaceutically acceptable polymer is vinylpyrrolidone-vinylacetate copolymer, polyethylene glycol-polyvinylcaprolactam-polyvinylacetate copolymer, or a mixture thereof.

2. The composition of Claim 1, wherein the polymer comprises vinylpyrrolidone-vinylacetate copolymer.

3. The composition of Claim 1, wherein content (weight) ratio of the rivaroxaban and the polymer is 1:0.5 to 1:5 (rivaroxaban : total polymer content).

4. The composition of Claim 1, wherein the melt extrusion is conducted at 160 to 230°C.

5. The composition of Claim 4, wherein the melt extrusion is conducted at 180 to 210°C.

6. The composition of Claim 1, wherein the composition comprises a surfactant in an amount of 30 weight% or lower based on the rivaroxaban and the total polymer weight.

7. The composition of Claim 1, wherein 70% or more of the rivaroxaban is released within 2 hours at a dissolution test condition of rotating a paddle at 75 rpm and using 900 ml of water as a dissolution media.

8. The composition of Claim 1, wherein the rivaroxaban contained in the composition has solubility of 18 µg/ml or higher based on 37°C distilled water.

9. A method for manufacturing a composition comprising rivaroxaban in the amorphous or thermodynamically metastable crystal form, which comprises the steps of:
(S1) mixing (a) the rivaroxaban and (b) a polymer selected from vinylpyrrolidone-vinylacetate copolymer, polyethylene glycol-polyvinylcaprolactam-polyvinylacetate copolymer, or a mixture thereof, and
(S2) melt extruding the mixture.
